# EUROPEAN PATENT SPECIFICATION

(11) **EP 3 018 600 B1**
(45) Date of publication and mention of the grant of the patent: **29.12.2021**
(21) Application number: 14192494.4
(22) Date of filing: 10.11.2014
(51) Int. Cl.: G16H 40/63

(54) **Blood purification device user interface feedback method**
Feedbackverfahren für die Benutzeroberfläche einer Blutreinigungsvorrichtung
Procédé de rétroaction de dispositif de purification du sang

(43) Date of publication of application: 11.05.2016
(73) Proprietor: B. Braun Avitum AG, 34212 Melsungen (DE)
(72) Inventor: Bocz, Máté, 1094 Budapest (HU); Béky, Zsófia, 1037 Budapest (HU)
(74) Representative: Winter, Brandl - Partnerschaft mbB

(56) References cited:
- US-A- 6 104 397
- US-A1- 2004 233 201
- US-A1- 2012 138 533
- US-A1- 2014 325 447
- US-A1- 2015 227 293
- US-B1- 7 719 542

## Description

The invention relates generally to blood purification and renal replacement therapy. More specifically, the invention relates to monitoring device states during blood purification and renal replacement therapy, and to a generation of feedback informing a user on the state of a currently triggered event in a device.

Blood purification devices are complex systems, combinations of hardware and software components. The operator of such machines is often not aware of the status of these components for various reasons, for example because they don't see them or they don't recognize them. Operators need to know whether the blood and fluid pumps are working and are delivering the set flow rate, or whether the drug for anticoagulation is being applied to the patient. Devices often employ solutions on the graphical user interface to give feedback to the operator on the status of the device elements. They can be in forms of icons, sound, animations, and the like.

Among known methods and systems, a device may be designed for controlling parameter values to enable a user to quickly and easily control variable parameter values using a graphical user interface and to enable the user to obtain the desired precision of control of such variables. One such system provides four control functions: a data entry function, single step function, a scroll function, and a translation function. The user can select the particular control function used to control a parameter value in light of the particular amount or type of control that needs to be accomplished.

The US 6 104 397 A discloses a method and system for generating improved command progress indicator by establishing at a location of a display at least one computer-generated graphic indicator associated with at least one computer executable operation in a first-state appearance, the graphic indicator having a first-state appearance and a dynamic second-state appearance, signaling the computer to conduct an operation, altering the appearance of the computer-generated graphic indicator from the first-state appearance to the dynamic second-state appearance while the computer is conducting the operation, and restoring the appearance of the computer-generated graphic indicator from the dynamic second-state appearance to the first-state appearance when the computer has completed the operation.

The US 2014/325447 A1 discloses a method for displaying an icon in a terminal device and the terminal device thereof. The method includes monitoring an implementation of an event which controls a display of the icon; determining animation display parameters according to properties of the icon, if the implementation of the event which controls the display of the icon is detected; and controlling the icon to be displayed in an animated manner in accordance with the animation display parameters.

WO2014/033119 discloses an improved user interface approach for dialysis apparatuses.

Another known medical apparatus comprises a user interface for setting parameters and includes a screen for visualizing values of said parameters, a main control unit connected to the interface, a first memory and a video memory both connected to the main control unit for storing data corresponding to images on screen. The main control unit allows setting of a new value for a parameter, displays the new value on a screen region, stores the new value in the first memory, captures from the video memory data representative of said screen region, and verifies from said representative data if the displayed value corresponds to the value in the first memory.

The known arrangements of such kind are, therefore, generally about setting parameters, related to a safety check of parameters entered by the user, lighting of hardware buttons, and the like and do not motivate any suitable concept of improving the awareness and recognition of states and processes in a blood purification device with a view to improved user confidence and usability.

In view of the above, an object of the invention resides in providing a blood purification device and method therefor that are arranged to give improved and unambiguous feedback on an active user interface element regarding the status of actuators associated with that active element.

In addition, the invention shall provide a method for increasing user feedback on a graphical user interface on what is happening with parts of the device as a consequence of interacting with a graphical user interface element thereof.

According to the invention, this object is accomplished by a method for providing feedback in a blood purification device as defined in claim 1 and by a blood purification device arranged to carry out the method as defined in claim 8.

Advantageous further developments of the invention are subject of the accompanying dependent claims. According to a basic concept underlying the invention, a graphical user interface consists of various elements, such as buttons, icons, boxes, values, texts and so on. A button is an active element, meaning that the operator can interact with it by pressing it. This action triggers an event within the device. An animated feature is added to the button in order to catch a users' attention and to direct it to the button, and to provide feedback on the consequence of pressing the button, to inform the user about the state of the device element which was triggered by the button. The button and the animated element are connected such that the animation is placed on the button (meaning that if the animation is pressed, the button is pressed as well, and this way the animation and the button are integrated), and that the button and the animation both relate to the same device function (the button triggers an event for this function and the animation gives feedback on the status). Core features of the method described herein are directed to the control linking machine functions, events and states toward generating respective feedback on the active user interface element, and a key functionality is to give unambiguous information when needed.

Advantages of the invention result in at least that an operator is aware of the status of the fluid delivery process, in improved user recognition of the system status, in increased user confidence while using the device, and in improved quality of the human-computer interaction, all together yielding enhanced device usability.

Thus, according to an aspect of the invention, the object is accomplished by a method for providing animated feedback on an event state in a blood purification device, comprising the steps of: detecting a pressed state of an active element on a graphical user interface of the device; triggering an event in the device in response to the detection of the pressed state of the active element; tracking the status of the triggered event, wherein said tracking includes detecting a plurality of states of the event, and wherein each detected event state is assigned a feedback representation variant at the active element; and setting the feedback representation variant at the active element as a user feedback indication in accordance with the detected event state.

Preferably, each feedback representation variant assigned to a detected event state is set at the active element, at which the event was triggered, in a manner surrounding the active element. This is advantageous in that a surrounding feedback variant or animation thereof leaves enough button space for e.g. additional design, labeling, and the like. It is, however, understood that there is no limitation to a surrounding arrangement, and the feedback variant can also be overlaid onto the button area itself.

Preferably, each feedback representation variant assigned to a detected event state is set at the active element, at which the event was triggered, in a manner variably surrounding the active element in color, thickness and/or angle of an opacity gradient. Differences in appearance and/or variability thereof help catching the user's attention and provide for easy distinction of plural states fed back to a same button.

Feedback representation variants are grouped according to basic appearance, and animation of the basic appearance in a group is controlled by setting at least one of animation parameters upon detection of each event state and alteration of the basic appearance in accordance with a detection of a changed event state and a set at least one animation parameter. By grouping by basic appearance, such as colored outlines of the same thickness, thicker and thinner outlines of the same color, or same color and thickness but different angles of gradient, and controlling variations and/or animations by setting respective parameters defining a current appearance, it is possible to combine several feedback representation variant groups at a same button and e.g. switch them on and off, and change them, case, state and/or application dependent. On the one hand, a number of states representable at a same button can be increased in this way, and on the other hand, it is possible to support a basic graphical user interface by both hardware and software, but have it look differently between devices.

In this respect, it is advantageous when said at least one animation parameter includes at least a color parameter defining the color in which an active element surrounding frame appears and/or or color-pairs the gradient uses as a starting and ending gradient color, a speed parameter defining the speed of a feedback representation variant animation, a border weight parameter defining a thickness of the active element surrounding frame, a size parameter setting the size of an outline of the feedback representation variant animation, and/or an enable parameter controlling a visibility of the feedback representation variant animation.

As mentioned above, providing steps for setting multiple feedback representation variant groups to the same active element is advantageous in some aspects.

In addition preferably, spare representation variants not assigned to a currently triggered event are kept available for use in an escalating step enhancing the set feedback representation variant beyond normal in case of an exceptional event state. In practice, unforeseen exceptions such as emergency halt conditions, defective components, unstable controls and immediate alert requirements not stemming from the currently triggered event and/or its states may occur. Being able to overdrive regular control and feedback representation is advantageous in such cases to help catching the user's immediate attention.

As well preferably, there is a further step of resetting the feedback representation at the active element upon detection of a completed state of the triggered event. Thereby, any animation of the feedback representation is also halted, and the user can easily know that the event is terminated or completed, and/or recognize that no event is actively processed.

According to another aspect of the invention, a blood purification device comprising components providing trackable event states and a graphical user interface including one or more active elements arranged to trigger one or more events in the device and to present animated feedback of one or more tracked event states to a user of the device is arranged to carry out the method according to one of the preceding steps. Advantageously, the method can be operated on any blood purification or renal therapy device or machine equipped with supporting hardware and arranged to carry out the method.

Hereinafter, the invention will be described in more detail based on a preferred embodiment thereof and with reference to the attached drawings. In the drawings,

Fig. 1 schematically shows a flow chart of an operation flow in a blood purification device controlling user feedback at an active element in a graphical user interface of the device according to a preferred embodiment; and

Fig. 2 schematically exemplifies feedback presentations to a user according to the control carried out in the embodiment.

Generally referring to the preferred embodiment, the configuration thereof basically relies on supporting hardware provided in any blood purification device/machine or blood purification related device benefiting from the embodied blood purification device feedback method. Such hardware can, therefore, in particular comprise sensors, detectors, pumps, filters and various other process and measurement means of which a state or status can be queried, polled and/or sampled, or that deliver a certain value during device operation, and on suitable means arranged to process such queried status and/or values and generate an output based on the processing result.

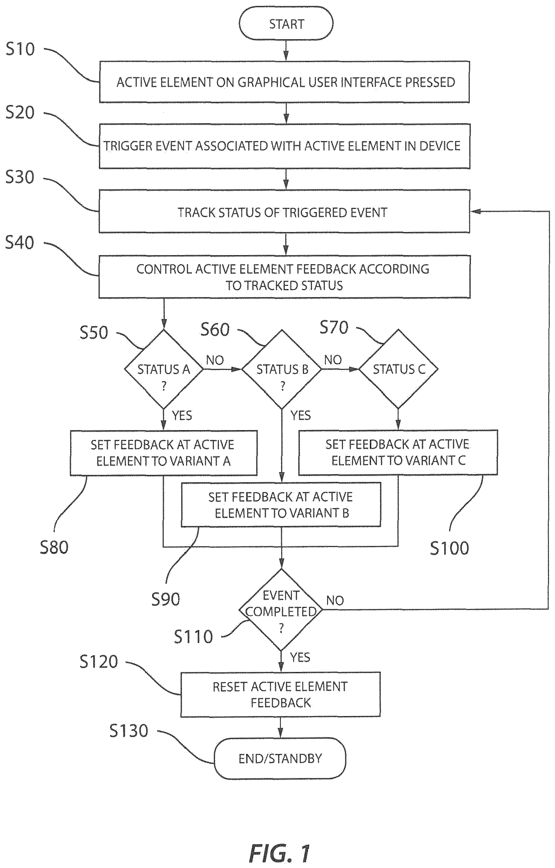

Fig. 1 schematically shows a flow chart of an operation flow in a blood purification device controlling user feedback at an active element in a graphical user interface of the device according to a preferred embodiment.

After the control is initiated, in a step S10, an active element on a graphical user interface of a blood purification device is pressed or touched by a user. Accordingly, the active element can preferably be a representation of a button or key.

In a step S20, an event associated with the active element is triggered in the blood purification device. The triggered event can be any function controllable by the pressing of the active element, any may include, without being limited thereto, e.g. supplying and/or disconnecting a component with/from power, starting, stopping and/or moving of mechanically linked or coupled components and/or actuators, and/or calling software routines which then take over control and may trigger other events. A particularly applicable device function may be such that it can take several states after having been triggered and before it is terminated, and the several states may then be presented to the user as different feedback states of the active element associated therewith.

In a following step S30, the status of the triggered event is tracked internally in the device. Status tracking is done by some suitable processing environment provided in the device and as such not subject of the present disclosure.

The tracked status is rendered in suitable form to a next step S40, in which the user feedback at the associated active element is controlled according the tracked status. A tracked status in a blood purification device may, for example, relate to a pump speed and/or amount of a pump involved in a fluid delivery process, without being limited thereto.

In this example case, the pump speed may, for example, a predetermined minimum, a predetermined maximum, and an intermediate speed between the minimum and the maximum speed, or a number of predetermined selectable speeds. In other word, the function, device element or actuator assigned to the active element in the graphical user interface may have a number N of distinguishable states. In the present embodiment, three states A, B and C are exemplified representative of the number N.

Given the example three states A, B and C, step S40 then develops into a step S50, a step S60 and a step S70, in which it is checked whether a currently tracked status is a state or status A, a state or status B, or a state or status C. Depending on the checking result obtained in steps S50 to S70, the active element is set to a user feedback variant A in a step S80, to a user feedback variant B in a step S90, or to a user feedback variant C in a step S100.

Generally, a number of available feedback variants at the active element may correspond to the number of states the assigned device element can take. It is, however, also possible to additionally provide sort of "spare" or "very important" variants, which may be useful for example in cases where non-predicted, disallowed and/or non-trackable states must be intercepted, or where some additionally fed input, maybe an emergency or safety related signal or event, is intended to quickly escalate the as such regular feedback in order to catch the user's immediate attention.

After the currently applicable feedback has been set to the active element in one of the steps S80 to S100, the process proceeds to a step S110. In step S110, it is confirmed whether or not the initially triggered event has been completed. A completed event may be taken as finalized, so that no more states are to be tracked, and control of the active element can be halted for the time being.

If the event is completed, the process proceeds to a step S120, in which the active element and its indicated feedback, respectively, is reset to a standard configuration. The standard configuration may in particular correspond to a non-pressed state of the active element, similar to an idle state from which pressing the active element by the user again would start the flow anew and call step S10.

If it is found in step S110 that the event is not completed, the process returns to step S30, in which the tracking of the status of the triggered event is continued. As long as the event is not completed, steps S50 to S110 are subsequently looped in order to adapt the user feedback at the active element to any state change having occurred since the last pass. It is noted that such state changes may occur self-triggered, for example if a device control becomes unstable and causes deviations elsewhere, or as retriggered by the active element prior to the event being completed. In the latter case, it is understood that the active element may comprise a manual restart and/or halt function and/or a multi-step (up/down) functionality in a way that manipulating the active element after its first depression actively changes the event state.

Step S120 is followed by a step S130, which is an end step or return step in which the process returns to a state in which it can be called again by pressing an active element on the graphical user interface.

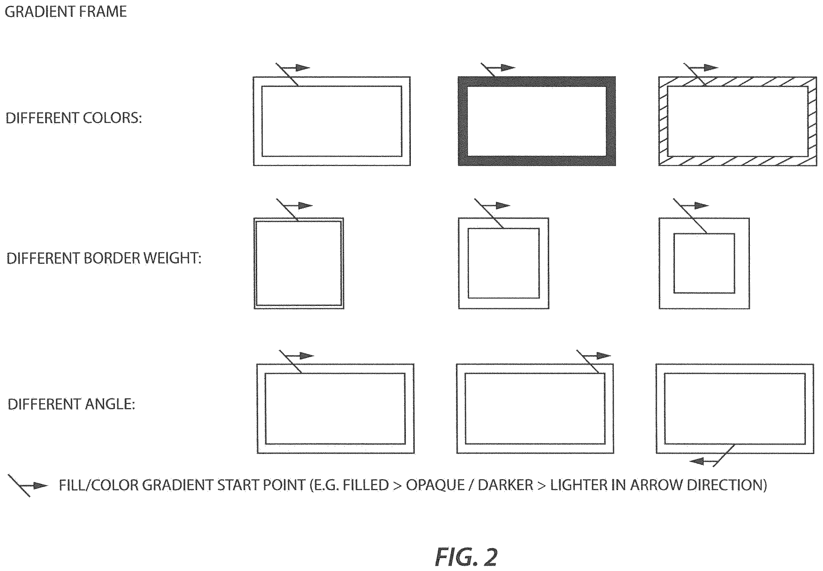

Turning to Fig. 2, feedback presentations to a user according to the control carried out in the embodiment are schematically exemplified based on a rectangle as an example. It is, however, understood that various representations other than a rectangle are possible.

In the present preferred embodiment, an animation is placed on or around the button as the active element. The animation follows the outline of the button with the sides being colored (with colors not represented as such in the figure) and/or animated, and with the middle being transparent so that the button can be seen.

According to Fig. 2, examples of various possible forms, or variants, for this animation include e.g. a gradient colored frame with rotating angle of gradient with fixed border weight, a same shape of the animation and the button itself, but the animation is semi-transparent and is changing its color and/or its pattern, a same shape of the animation and the button itself, but the animation is semi-transparent and creates a visual illusion as if the button was being pushed and released in 3D, and the like.

In order to control the animation to represent the various forms, or variants, a basic set of animation parameters may be selected in step S40, and variants thereof may be set state dependent in steps S80 to S100. In other words, step S40 may select a basic appearance of the animation, and steps S80 to S100 may change the basic appearance depending on the determined state. Alternatively, step S40 may include preparatory functions, and steps S80 to S100 may be in full control of the animation to be set and output.

Thereby, as example animation parameters and without limitation thereto, a color parameter defines the color in which a frame appears or what color-pairs the gradient uses as a starting and ending gradient color. A speed parameter can define the speed of the animation. When set to 0, the frame is static, and when set to other than 0, the frame is either rotating the angle of gradient or repeatedly changes an opacity value of the color from 0 to 100 and back in order to provide a blinking effect. A border weight parameter defines the number of pixels of the thickness of the frame. A size parameter sets the size of the outline (e.g. a rectangle, a circle, an oval of the like) of the animation. An enable parameter is used to control the visibility of the animation. When the enable parameter is set to 1, the animation is active and visible, and when it is set to 0, the animation is hidden.

While the invention has been described with reference to a preferred embodiment and the accompanying drawings, it is understood that the present invention is not in any way limited to particular details disclosed with respect to this preferred embodiment, and that any modification readily apparent to the skilled person based on the here presented teaching is deemed to be within the scope of protection as defined by the appended claims.

## Claims

1. A method for providing animated feedback on an event state in a blood purification device, comprising the steps of:
detecting a pressed state of an active element on a graphical user interface of the device;
triggering (S20) an event in the device in response to the detection of the pressed state of the active element;
tracking (S30) the status of the triggered event, wherein said tracking includes detecting a state of the event out of a set of a plurality of distinguishable event states by detecting the status of hardware provided in the blood purification device comprising process and measurement means, in particular sensors, detectors, pumps and/or filters, and wherein each of the distinguishable event states is assigned a feedback representation variant at the active element;
setting (S40) the feedback representation variant at the active element as a user feedback indication in accordance with the detected event state; **characterised in that** feedback representation variants are grouped according to basic appearance, and animation of the basic appearance in a group is controlled by setting at least one of animation parameters upon detection of each event state and alteration of the basic appearance in accordance with a detection of a changed event state and a set at least one animation parameter.

2. A method according to claim 1, wherein each feedback representation variant assigned to a detected event state is set at the active element, at which the event was triggered, in a manner surrounding the active element.

3. A method according to claim 1 or 2, wherein each feedback representation variant assigned to a detected event state is set at the active element, at which the event was triggered, in a manner variably surrounding the active element in color, thickness and/or angle of an opacity gradient.

4. A method according to claim 1, wherein said at least one animation parameter includes a color parameter defining the color in which an active element surrounding frame appears and/or or color-pairs the gradient uses as a starting and ending gradient color, a speed parameter defining the speed of a feedback representation variant animation, a border weight parameter defining a thickness of the active element surrounding frame, a size parameter setting the size of an outline of the feedback representation variant animation, and/or an enable parameter controlling a visibility of the feedback representation variant animation.

5. A method according to claim 1, wherein multiple feedback representation variant groups are settable to the same active element.

6. A method according to one of the preceding claims, wherein spare representation variants not assigned to a currently triggered event are kept available for use in an escalating step enhancing the set feedback representation variant beyond normal in case of an exceptional event state.

7. A method according to one of the preceding claims, comprising the step of resetting the feedback representation at the active element upon detection of a completed state of the triggered event.

8. A blood purification device comprising components providing a plurality of distinguishable and trackable event states and a graphical user interface including an active element arranged to trigger an event in the device and to present animated feedback of the plurality of distinguishable and trackable event states to a user of the device by detecting the status of hardware provided in the blood purification device comprising process and measurement means, in particular sensors, detectors, pumps and/or filters, the blood purification device being arranged to carry out the method according to one of the preceding claims.

## Patentansprüche

1. Verfahren zur Bereitstellung einer animierten Rückmeldung über einen Ereigniszustand in einer Blutreinigungsvorrichtung, mit den folgenden Schritten:
Erfassen eines gedrückten Zustands eines aktiven Elements auf einer graphischen Benutzeroberfläche der Vorrichtung;
Auslösen (S20) eines Ereignisses in der Vorrichtung in Reaktion auf die Erfassung des gedrückten Elements;
Verfolgen (S30) des Zustands des ausgelösten Ereignisses, wobei das Verfolgen das Erfassen eines Zustands des Ereignisses aus einem Satz von einer Vielzahl von unterscheidbaren Ereigniszuständen durch Erfassen des Zustands von Hardware umfasst, die in der Blutreinigungsvorrichtung mit Prozess- und Messmittel, insbesondere Sensoren, Detektoren, Pumpen und/oder Filter, vorgesehen ist, und wobei jedem der unterscheidbaren Ereigniszustände eine Rückkopplungsdarstellungsvariante an dem aktiven Element zugeordnet ist;
Einstellen (S40) der Rückmeldungsdarstellungsvariante an dem aktiven Element als Benutzerrückmeldungsanzeige entsprechend dem erfassten Ereigniszustand; **dadurch gekennzeichnet, dass**
die Rückkopplungsdarstellungsvarianten entsprechend dem Grunderscheinungsbild gruppiert sind und die Animation des Grunderscheinungsbildes in einer Gruppe durch Einstellen von mindestens einem von Animationsparametern bei Erfassung jedes Ereigniszustandes und Änderung des Grunderscheinungsbildes in Übereinstimmung mit einer Erfassung eines geänderten Ereigniszustandes und einer Einstellung zumindest eines Animationsparameters gesteuert wird.

2. Verfahren gemäß Anspruch 1, wobei jede einem erfassten Ereigniszustand zugeordnete Rückkopplungsdarstellungsvariante an dem aktiven Element, an welchem das Ereignis ausgelöst wurde, in einer das aktive Element umgebenden Weise gesetzt wird.

3. Verfahren gemäß einem der Ansprüche 1 oder 2, wobei jede einem erfassten Ereigniszustand zugeordnete Rückkopplungsdarstellungsvariante an dem aktiven Element, an welchem das Ereignis ausgelöst wurde, in einer das aktive Element variabel umgebenden Weise in Farbe, Dicke und/oder Winkel eines Opazitätsgradienten eingestellt wird.

4. Verfahren nach Anspruch 1, wobei dieser zumindest eine Animationsparameter einen Farbparameter hat, der eine Farbe definiert, in welcher ein aktives Element, das den Rahmen umgibt, erscheint, und/oder Farbpaare, die der Gradient als Start- und Endgradientenfarbe verwendet, einen Geschwindigkeitsparameter, der die Geschwindigkeit einer Animation mit Rückkopplungsdarstellung definiert, einen Randgewichtsparameter, der die Dicke des aktiven Elements, das den Rahmen umgibt definiert, einen Größenparameter, der die Größe eines Umrisses der Animation mit Rückkopplungsdarstellung festlegt, und/oder einen Freigabeparameter, der die Sichtbarkeit der Animation mit Rückkopplungsdarstellung steuert.

5. Verfahren gemäß Anspruch 1, wobei mehrere Gruppen der Rückkopplungsdarstellungsvarianten auf dasselbe aktive Element einstellbar sind.

6. Verfahren gemäß einem der vorhergehenden Ansprüche, wobei Ersatzdarstellungsvarianten, die einem aktuell ausgelösten Ereignis nicht zugeordnet sind, zur Verwendung in einem Eskalationsschritt verfügbar gehalten werden, der die eingestellte Rückkopplungsdarstellungsvariante im Falle eines außergewöhnlichen Ereigniszustands über das Normale hinaus erweitert.

7. Verfahren gemäß einem der vorhergehenden Ansprüche, mit dem Schritt des Zurücksetzens der Rückkopplungsdarstellung an dem aktiven Element bei Erfassung eines abgeschlossenen Zustands des ausgelösten Ereignisses.

8. Blutreinigungsvorrichtung mit Komponenten, die eine Vielzahl von unterscheidbaren und verfolgbaren Ereigniszuständen bereitstellen, und eine graphische Benutzerschnittstelle, die ein aktives Element hat, dass so ausgebildet ist, um ein Ereignis in der Vorrichtung auszulösen und einem Benutzer der Vorrichtung eine animierte Rückmeldung der Vielzahl von unterscheidbaren und verfolgbaren Ereigniszuständen präsentiert, indem in der Blutreinigungsvorrichtung vorgesehene Hardware mit Prozess- und Messmittel, insbesondere Sensoren, Detektoren, Pumpen und/oder Filter erfasst wird, wobei die Blutreinigungsvorrichtung so beschaffen ist, um das Verfahren nach einem der vorhergehenden Ansprüche auszuführen.

## Revendications

1. Procédé de fourniture d'un retour animé sur un état d'événement dans un dispositif de purification du sang, comprenant les étapes consistant à :
détecter un état d'appui d'un élément actif sur une interface utilisateur graphique du dispositif ;
déclencher (S20) un événement dans le dispositif en réponse à la détection de l'état d'appui de l'élément actif ;
suivre (S30) l'état de l'événement déclenché, dans lequel ledit suivi inclut une détection d'un état de l'événement parmi un ensemble d'une pluralité d'états d'événement pouvant être distingués en détectant l'état de matériel fourni dans le dispositif de purification du sang comprenant des moyens de processus et de mesure, en particulier des capteurs, des détecteurs, des pompes et/ou des filtres, et dans lequel à chacun des états d'événement pouvant être distingués est attribué une variante de représentation de retour au niveau de l'élément actif ;
régler (S40) la variante de représentation de retour au niveau de l'élément actif en tant qu'indication de retour utilisateur conformément à l'état d'événement détecté ; **caractérisé en ce que** les variantes de représentation de retour sont groupées conformément à un aspect de base, et une animation de l'aspect de base dans un groupe est commandée en réglant au moins un de paramètres d'animation lors de la détection de chaque état d'événement et d'une modification de l'aspect de base conformément à une détection d'un état d'événement changé et à un ensemble d'au moins un paramètre d'animation.

2. Procédé selon la revendication 1, dans lequel chaque variante de représentation de retour attribuée à un état d'événement détecté est réglée au niveau de l'élément actif, au niveau duquel l'événement a été déclenché, d'une manière entourant l'élément actif.

3. Procédé selon la revendication 1 ou 2, dans lequel chaque variante de représentation de retour attribuée à un état d'événement détecté est réglée au niveau de l'élément actif, au niveau duquel l'événement a été déclenché, d'une manière entourant de façon variable l'élément actif en couleur, épaisseur et/ou angle d'un gradient d'opacité.

4. Procédé selon la revendication 1, dans lequel ledit au moins un paramètre d'animation inclut un paramètre de couleur définissant la couleur dans laquelle un cadre entourant un élément actif apparaît et/ou apparie en couleur les utilisations de gradient en tant que couleur à gradient de début et de fin, un paramètre de vitesse définissant la vitesse d'une animation de variante de représentation de retour, un paramètre de poids de bordure définissant une épaisseur du cadre entourant un élément actif, un paramètre de taille réglant la taille d'un contour de l'animation de variante de représentation de retour, et/ou un paramètre d'activation commandant une visibilité de l'animation de variante de représentation de retour.

5. Procédé selon la revendication 1, dans lequel de multiples groupes de variantes de représentation de retour sont réglables sur le même élément actif.

6. Procédé selon l'une des revendications précédentes, dans lequel des variantes de représentation restantes non attribuées à un événement déclenché couramment restent à disposition pour une utilisation dans une étape d'intensification mettant en valeur la variante de représentation de retour réglée au-delà de la normale dans le cas d'un état d'événement exceptionnel.

7. Procédé selon l'une des revendications précédentes, comprenant l'étape consistant à réinitialiser la représentation de retour au niveau de l'élément actif lors de la détection d'un état terminé de l'événement déclenché.

8. Dispositif de purification du sang comprenant des composants fournissant une pluralité d'états d'événement pouvant être distingués et pouvant être suivis et une interface utilisateur graphique incluant un élément actif agencé pour déclencher un événement dans le dispositif et pour présenter un retour animé de la pluralité d'états d'événement pouvant être distingués et pouvant être suivis à un utilisateur du dispositif en détectant l'état de matériel fourni dans le dispositif de purification du sang comprenant des moyens de processus et de mesure, en particulier des capteurs, des détecteurs, des pompes et/ou des filtres, le dispositif de purification du sang étant agencé pour réaliser le procédé selon l'une des revendications précédentes.
